# EUROPEAN PATENT APPLICATION

(11) **EP 1 035 213 A1**
(43) Date of publication of application: **13.09.2000**
(21) Application number: 98950496.4
(22) Date of filing: 02.11.1998
(51) Int. Cl.: C12Q 1/37, C12Q 1/25

(54) **METHOD FOR ENZYME ASSAY**

(30) Priority: 04.11.1997 JP 30161497
(71) Applicant: FUJI PHOTO FILM CO., LTD., Kanagawa-ken, 250-0193 (JP)
(72) Inventor: NISHIGAKI, Junji Fuji Photo Film Co., Ltd.,, Minami-ashigara-shi Kanagawa 250-0193 (JP); TAMURA, Yutaka Fuji Photo Film Co., Ltd.,, Minami-ashigara-shi Kanagawa 250-0193 (JP); HAMAOKA, Tsutomu, Hadano-shi Kanagawa 257-0011 (JP)
(74) Representative: HOFFMANN - EITLE
(86) International application number: JP9804958
(87) International publication number: WO9923245

(57) **Abstract**

A method of assaying enzymes such as protease, comprising the step (1) of bringing an enzyme-containing specimen into contact with a thin film containing both of an organic compound having a visible ray absorption that varies by the reaction with an enzyme and a hydrophilic macromolecule such as gelatine and the step (2) of detecting discernible traces formed on the film by the action of the enzyme.

## Description

### Technical Field

The present invention relates to a method for measuring an enzyme such as proteases. More specifically, it relates to a method for measuring an enzyme such as proteases which enables accurate diagnosis of malignancy of cancer cells such as infiltrative and metastatic activity, degree of progress of periodontal diseases such as alveolar pyorrhea, destructive pathological conditions in rheumatoid arthritis and the like. The present invention further relates to a thin membrane used for the measurement of an enzyme and a compound used for producing the thin membrane.

### Related Art

Presence or absence of infiltration into interstitial tissue is one of the factors that determine difference of benignancy and malignancy of tumor. In order to clarify the pathological conditions, it is necessary to observe changes of growth dynamics of tumor cells, per se, and to find factors effecting the interaction between tumor cells and interstitial tissues. In particular, it has been revealed that proteases are involved in infiltration and metastasis of tumor cells, and accordingly, the inhibition of infiltration and metastasis of malignant tumor cells is expected by controlling proteases. Among such proteases (extracellular matrix lyases), important roles of matrix metalloproteinase (MMP), in particular, has been elucidated in the growth and infiltration of cancer cells, and arterialization (see, "Molecular Mechanism of Tumor Metastasis", Ed. By T. Tsuruo, Chapter 8; K. Miyazaki, "Matrix Proteases and Infiltration/metastasis of Cancer", pp. 92-107, Medical View Co., Ltd., 1993).

Periodontal diseases progress with destruction as early nidus of crevicular epithelium and connective tissues which mainly consist of collagen, and the involvement of matrix metalloproteinase has also been known in the destruction of tissues (for the involvement of proteases in the destruction of periodontal tissues and correlation of pathological conditions and proteases, see "Science of Periodontal Treatment", Ed. by M. Aono, Chapter VII, M. Shirakawa, "Pathology of Periodontal Tissues", pp.99-109, Ishiyaku Shuppan; Hasegawa et al., "Gelatinase Activity in Gingival Crevicular Fluids (GCF) of Periodontal Disease Patients", Presentation No. A-44, 37th Fall Congress of Japan Periodontal Disease Society and other).

The matrix metalloproteinases degrade extracellular substrates such as collagen, proteoglycan, laminin, fibronectin and gelatin, and the existence of eight types, e.g., MMP-1, 2, 3, 7, 9, 10, are known. Interstitial collagenase (MMP-1), a matrix metalloproteinase known for the longest, distributes over fibroblasts, cartilage and the like, and cleaves interstitial collagen into 1/4 and 1/3 portions. In periodontal diseases, mainly MMP-2 (gelatinase A) and MMP-9 (gelatinase B) destroy, for example, collagen type IV, laminin, fibronectin, and proteoglycan as components of periodontal tissues. The secretion of matrix metalloproteinase is strongly promoted by EGF and TGF-β as cell growth factors, and the secretion and the expression of activity are controlled by endogenous inhibitors in tissues. However, the ways of suppression of its expression have not been fully clarified when growth factors are involved.

An example of another protease involved in infiltration and metastasis of tumor cells includes plasminogen activator (PA) as one of serine proteases. The plasminogen activator converts plasminogen into plasmin, and plasmin formed by the action of plasminogen activator converts a prometalloproteinase into a metalloproteinase as an active form. Accordingly, it is considered that infiltration and metastasis of cancer cells are progressed or accelerated by a cascade formed between matrix metalloproteinases and plasminogen activator.

Protease may possibly participate in destructive pathological conditions such as destruction of bone tissue and alveolar periosteum caused by alveolar pyorrhea and destruction of periost and bone tissue caused by rheumatoid arthritis, as well as the infiltration and metastasis of cancer cells and progress of periodontal diseases (as for the involvement of proteases in rheumatism, see, Nippon Rinsho [Japan Clinic], 50(3), pp.463-467, 1992). Therefore, quantitative measurement of protease in cells and tissues may enable accurate diagnosis of malignancy of cancer cells on the basis of infiltrative and metastatic activity, pathological condition of periodontal diseases, and degree of progress of destructive pathological conditions such as rheumatism (as for correlation between levels of infiltration of cancer cells and protease activity, see, for example, Yamagata, et al., Cancer Lett., 59, 51, 1991; Azzam, et al., J. Natl., Cancer Inst., 85, 1758, 1993; Brown, et al., Clin. Exp. Metastasis, 11, 183, 1993; Davies, et al., Br. J. Cancer, 67, 1126, 1993).

As methods for measuring protease, zymography methods where enzyme activity is determined based on the degree of substrate degradation, immunoblotting methods utilizing antibodies specific for each protease or the like have been used. For example, a method is known which comprises the steps of homogenizing cancer cells or periodontosis cells, subjecting the extract to electrophoresis utilizing gelatin-containing SDS-polyacrylamide gel, staining the gel after the electrophoresis using Amido Black, followed by determining a sample as protease positive that provides a white transparent band not stained. However, according to the above method, the preparation of SDS-polyacrylamide gel is required for every measurement and the process takes about 30 hours before obtaining measuring results.

Another method is available where a gel after an electrophoresis according to the SDS-PAGE technique is placed on a membrane, and after blotting procedure, enzymes are detected by using monoclonal antibodies. However, the method also uses electrophoresis, which is the same drawback as the aforementioned method. In addition, this method has further problems that it requires skills for operation and uses expensive monoclonal antibodies. Furthermore, these methods do not achieve measurement of protease in each individual cell, but they only measure total protease in the whole tissue. Therefore, they also have a problem in failing to provide information about infiltrative and metastatic activity of individual cancer cells.

While seeking means to solve these problems, the inventors of the present invention found that, when a slice of a tissue such as cancer tissue is brought into contact with a surface of a thin membrane containing a protease substrate such as gelatin together with a hardening agent, or when exudate collected from a morbid tissue such as a tissue of a periodontal disease is dropped onto the thin membrane, proteases contained in the sample digest the thin membrane and form traces of digestion on the surface of the thin membrane. They also found that proteases expressed in individual cells can be determined by preparing histopathological preparations and the above thin membrane preparations each by using one of continuous tissue slices, and then comparing the resulting preparations (PCT/JP97/0588, International Publication WO97/32035). These methods are characterized in that they are superior in reproducibility compared with the method utilizing a thin membrane containing agarose (The FASEB Journal, Vol.9, July, pp.974-980, 1995), and can achieve accurate measurement of protease activity in samples.

### Disclosure of the Invention

An object of the present invention is to provide a convenient and accurate method for measuring an enzyme, in particular, a method for measuring a protease. More specifically, the object of the present invention is to provide a method for measuring a protease which can achieve prompt and accurate determination of malignancy of cancer cells such as infiltrative and metastatic activities, pathological conditions of periodontal diseases and the like, degree of progress of destructive pathological conditions such as in rheumatism, and is useful for accurate diagnosis of, for example, prognosis of cancer, degree of progress of destructive pathological conditions. Another object of the present invention is to provide a method for measuring a protease which has the characteristic features mentioned above, and is capable of accurately measuring a protease derived from cancer cells or the like localized in test tissues.

The inventors of the present invention further conducted various studies to achieve the foregoing objects. As a result, they found that organic compounds existed which were featured to react with a functional group or a metal ion derived from an enzyme, particularly a protease, to change their visible absorption. In addition, they also found that, when a slice of tissue or exudate collected from a morbid tissue was brought into contact with a surface of a thin membrane containing the organic compound and a hydrophilic polymer, and optionally a hardening agent, visible traces were formed, and that the enzymatic activity, in particular, protease activity were very easily and quickly detectable by using the thin membrane. Furthermore, they found that proteases expressed in individual cells were determinable by preparing histopathological preparations and thin membrane preparations each by using one of continuous tissue slices, or alternatively, by preparing a plurality of different thin membrane preparations, and then comparing the resulting preparations, and they also found that type of an enzyme such as a protease was accurately identifiable by using a multi-layered thin membrane comprising a plurality of different layers and comparing traces formed on each layer. The present invention was achieved on the basis of these findings.

The present invention thus provides a method for measuring an enzyme, which comprises the steps of (1) bringing a sample containing an enzyme into contact with a thin membrane comprising an organic compound which changes visible absorption upon reaction with the enzyme and a hydrophilic polymer; and (2) detecting a visible trace on the thin membrane formed by an action of the enzyme. The thin membrane may contain a hardening agent, and the enzyme to be measured is preferably a protease, and most preferably matrix metalloproteinase. The organic compound, which changes visible absorption upon reaction with an enzyme, preferably reacts with a thiol group and/or a metal ion (for example, zinc ion) in the enzyme. As the sample, a biosample may be used which is isolated or collected from a mammal including human, preferably a biosample selected from a cancer tissue slice, gingival crevicular exudate, or a destructive morbid tissue slice or extract. As methods for measuring an enzyme using the aforementioned thin membrane, the following methods are provided by the present invention.
(A) A method which comprises the steps of (1) bringing one of two substantially continuous slices of a biosample into contact with the aforementioned thin membrane; (2) detecting visible traces formed by action of the enzyme on the thin membrane; and (3) comparing the traces with a histopathological preparation prepared from the other slice;
(B) A method which comprises the steps of (1) bringing one of two or more substantially continuous slices of a biosample into contact with the aforementioned thin membrane; (2) bringing the remaining slice(s) into contact with one or more of the aforementioned thin membranes comprising an enzyme inhibitor; (3) detecting visible traces formed by action of the enzyme on each thin membrane; and (4) comparing the traces on the thin membrane used in the step (1) with the traces on the thin membrane(s) used in the step (2);
(C) A method which comprises the steps of (1) bringing one of several substantially continuous slices of a biosample into contact with the aforementioned thin membrane; (2) bringing the remaining slice(s) into contact with another one or more of the aforementioned thin membranes comprising an organic compound different from the organic compound contained in the thin membrane used in the step (1); (3) detecting visible traces formed by action of the enzyme on each thin membrane; and (4) comparing the traces on the thin membrane used in the step (1) with the traces on the thin membrane(s) used in the step (2);
(D) A method which comprises the steps of (1) bringing one of two or more substantially continuous slices of a biosample into contact with the aforementioned thin membrane; (2) bringing the remaining slice(s) into contact with another one or more of the aforementioned thin membranes comprising a substrate that is degradable by the enzyme; (3) detecting visible traces formed by action of the enzyme on each thin membrane; and (4) comparing the traces on the thin membrane used in the step (1) with the traces on the thin membrane(s) used in the step (2);
(E) A method which comprises the steps of (1) bringing a sample containing an enzyme into contact with a thin membrane comprising layer (a) which comprises one or more layers each comprising the aforementioned thin membrane and layer (b) which comprises one or more layers each comprising the aforementioned thin membrane further containing an enzyme inhibitor; (2) detecting visible traces formed by action of the enzyme on each thin membrane; and (3) comparing the traces on the layer (a) and the traces on the layer (b);
(F) A method which comprises the steps of (1) bringing a sample containing an enzyme into contact with a thin membrane comprising two or more layers each comprising a different aforementioned organic compound; (2) detecting visible traces formed by action of the enzyme on the thin membrane; and (3) comparing the traces on each of the layers; and
(G) A method which comprises the steps of (1) bringing a sample containing an enzyme into contact with a thin membrane comprising layer (a) comprising one or more layers each comprising the aforementioned thin membrane and layer (b) comprising one or mare layers each containing a substrate degradable by the enzyme; (2) detecting visible traces formed by action of the enzyme on the thin membrane; and (3) comparing the traces on the layer (a) and the traces on the layer (b).

Each of the thin membranes used for the above methods is preferably formed on a flat surface of a support, and an undercoat layer may be provided between the support and the thin membrane.

According to another aspect of the present invention, there are provided methods of diagnosing a disease in which an enzyme (preferably a protease, and most preferably matrix metalloproteinase) is involved, which comprises the steps of bringing a biosample isolated or collected from a patient (preferably selected from a cancer tissue slice, gingival crevicular exudate, or a destructive morbid tissue slice or extract) into contact with the aforementioned thin membrane (which may contain a hardening agent); and detecting visible traces formed on the thin membrane to measure activity of the enzyme in the biosample. The disease as the diagnostic object of the aforementioned method is preferably selected from the group consisting of cancers, rheumatic diseases, periodontal diseases, and alveolar pyorrhea. According to further aspects of the present invention, there are provided thin membranes used for the aforementioned methods, and novel organic compounds that can be used for the manufacture of the thin membranes.

### Best Mode for Carrying out the Invention

The methods for measurement of an enzyme of the present invention are characterized by comprising the steps of (1) bringing a sample containing an enzyme into contact with a thin membrane comprising an organic compound which changes visible absorption upon reaction with the enzyme and a hydrophilic polymer; and (2) detecting visible traces formed by the action of the enzyme on the thin membrane. The methods of the present invention are more excellent than the conventional method detecting traces of digestion (PCT/JP/0588, International Publication WO97/32035) in that (1) they achieve very high detection accuracy because the protease activity is detected on a molecular level, (2) they have very high detection sensitivity because of a high absorption coefficient, and (3) the operation is convenient because they do not require a staining step. The term "measurement" used in the present specification should be construed in its broadest sense including qualitative and quantitative measurements so as to encompass any measurements that can provide information on the existence of a biological substance such as an enzyme. The wording "visible traces formed on a thin membrane" according to the methods of the present invention and similar wordings thereto mean that a state is provided on the thin membrane in which a change in visible absorption can be detected as compared to the state of the thin membrane before measurement.

The enzymes as the object of the measurement according to the methods of the present invention are not particularly limited. Proteases are preferred, and matrix metalloproteinase (MMP) and matrix serine protease (MSP) are more preferred objects. These enzymes are explained in detail in "Molecular Mechanism of Cancer Metastasis", Ed. By T. Tsuruo, pp.92-107, Medical View Co., Ltd., 1993. Examples of proteases particularly suitable for the methods of the present invention include, for example, matrix metalloproteinases such as interstitial collagenase (MMP-1), gelatinase A (MMP-2) and gelatinase B (MMP-9); matrix serine proteases such as plasminogen activator (PA) and the like. However, the objects of the methods of the present invention are not limited to these enzymes.

As samples used for the methods of the present invention, for example, biosamples isolated or collected from mammals including human may be used. The biosamples may be tissues or tissue exudate and the like. For example, cancer tissues from solid tumor tissues of lung cancer, stomach cancer, esophageal cancer, breast cancer, brain tumor and the like which are isolated or collected by surgical operation or histological examination; synovial fluid and bone tissue of rheumatoid arthritis; destructive morbid tissues such as periodontal ligament or bone tissue and exudate of alveolar pyorrhea; gingival crevicular exudate of periodontal diseases and the like may be used.

When a tissue is used as a sample, for example, a slice having a thickness of 1-10 µm, preferably 5 µm may be prepared from a sample rapidly frozen in liquid nitrogen by using an apparatus for preparing frozen sections, and then the slice may be applied to a thin membrane to bring the sample contact with the thin membrane. When synovial fluid collected from a patient of rheumatoid arthritis is used as a sample, about 5 to 50 µl, preferably about 20 µl of synovial fluid can be dropped onto the thin membrane. When gingival crevicular fluid of periodontal disease is used as a sample, a piece of filter paper may be inserted into gingival crevice to collect about 5 to 10 µl of gingival crevicular fluid, and the filter paper may be applied to a thin membrane. After the collection of gingival crevicular fluid, the gingival crevicular fluid may be optionally extracted from the filter paper using distilled water or a suitable buffer (for example, 50 mM Tris-HCl, pH 7.5, 10 mM CaCl₂, 0.2 M NaCl), and the extract may be dropped onto a thin membrane.

The organic compounds used for the preparation of the thin membrane of the present invention are not particularly limited so long as a change in visible absorption occurs when they reacts with a functional group or a metal ion derived from an enzyme. The change in visible absorption caused by the reaction of an enzyme and the organic compound is not particularly limited, so long as significant difference between a reacted site and an unreacted site can be recognized by means of measurements of reflection of visible light or transmission density, absorption measurement and the like. As detection methods, density measurement or absorption measurement may preferably be used.

As functional groups of enzymes that react with the aforementioned organic compound, preferred examples include thiol group, thioether group, disulfide group, amino group (any types of amino group including aromatic, heterocyclic amino groups), hydroxy group, and carboxy group those present in amino acids as components of enzymes. A particularly preferred example includes thiol group. For detection of enzymes having a metal ion in an active center such as matrix metalloproteinases, it is preferable to use a compound that can form a stable complex with the metal ion. Detectable metal ions are not particularly limited. Zinc ion contained in matrix metalloproteinase is most preferred.

As compounds that react with thiol group, examples include those described in Bunseki Kagaku (Analytical Chemistry), Vol. 7, p.181 and Vol.37, p.421, and Anal. Sci., Vol.2, p.357, preferably those represented by the following formulas (2), (3), (4) and (5).

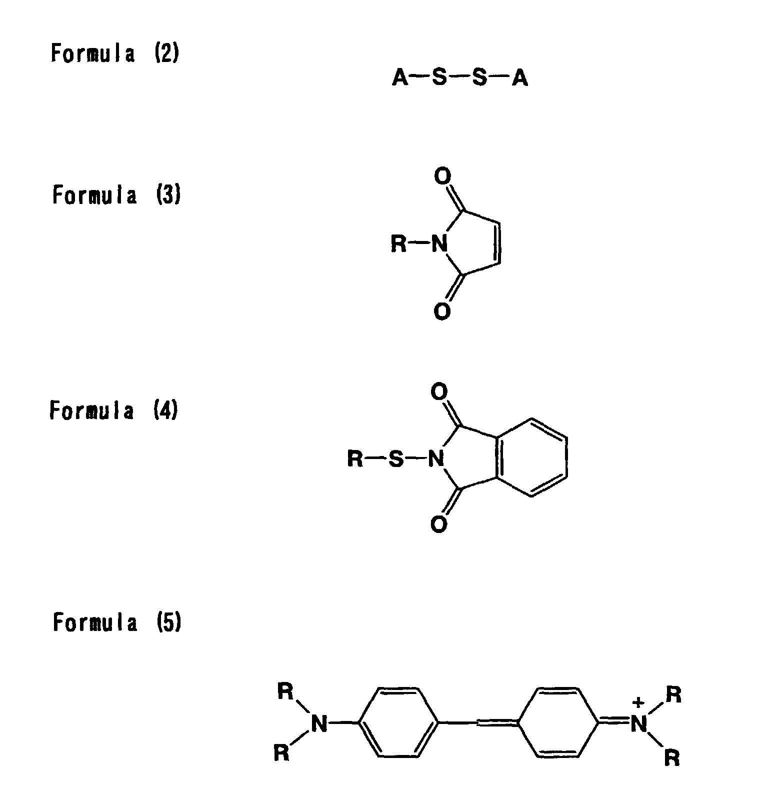

In the formulas, A independently represents an aryl group or a heteroaryl group, each of which may be substituted. Each of the group A may be the same or different. As the group A, a substituted or unsubstituted aryl group (for example, phenyl group) may preferably be used, and 4-amino-substituted phenyl group may more preferably be used. R independently represents hydrogen atom, an alkyl group (for example, a linear or branched alkyl group having 1 to 6 carbon atoms), an aryl group (for example, a substituted or unsubstituted phenyl group) or a heteroaryl group (for example, a substituted or unsubstituted pyridinyl group). Other substituents may further be bound to these substituents. As the group R, an aryl group or a heteroaryl group may preferably be used, and a phenyl group substituted with one or more electron withdrawing groups may more preferably be used. Symbol "n" represents 1 or 2, preferably 1.

As compounds that form a stable complex with a metal ion of an active center of an enzyme, examples include the cyanine-based compounds described in F.M. Hamer, "Heterocyclic Compounds - Cyanine Dyes and Related Compounds", John Wiley & Sons, New York, London (1964).

As compounds that form a stable complex with a metal ion of an active center of an enzyme, compounds represented by the following general formula (1) may be used wherein A independently represents an aryl group which may be substituted or a heteroaryl group which may be substituted, and n independently represents 1 or 2.

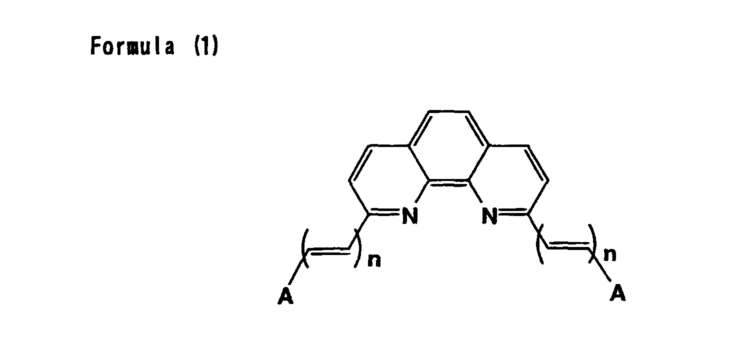

In the general formula (1), the two groups of A may be the same or different, and represent a substituted or unsubstituted aryl group or a substituted or unsubstituted heteroaryl group. As the aryl group, examples include those having 6 to 20 carbon atoms (for example, phenyl group, naphthyl group and the like). As heteroaryl compounds constituting the heteroaryl group, examples include pyridine, piperidine, triazine, pyrrole, imidazole, triazole, furan, thiophene, thiazole, oxazole, isothiazole, isoxazole and the like. The heteroaryl group may be constituted by a compound having a condensed ring formed by these heteroaryl compounds (for example, quinoline, benzimidazole, benzothiazole, benzoxazole and the like).

Those aryl groups and heteroaryl groups may have one or more substituents at any position(s). When they have two or more substituents, they may be the same or different. Examples of such substituents include, for example, a linear or branched alkyl group having 1 to 20 carbon atoms (for example, methyl group, ethyl group and the like); the above alkyl group which is substituted (for example, carboxymethyl group, sulfoethyl group and the like); a substituted or unsubstituted aralkyl group having 7 to 20 carbon atoms (for example, benzyl group, phenethyl group, p-methoxybenzyl group and the like); a linear or branched alkoxyl group having 1 to 8 carbon atoms (for example, methoxy group, ethoxy group and the like); a substituted or unsubstituted aryl group having 6 to 20 carbon atoms (phenyl group, naphthyl group, 4-sulfophenyl group, 2,5-disulfophenyl group, 4-carboxyphenyl group and the like); a substituted or unsubstituted aryloxy group having 6 to 20 carbon atoms (for example, phenoxy group, naphthoxy group, p-chlorophenoxy group and the like); a halogen atom (any of fluorine atom, chlorine atom, bromine atom, and iodine atom); carboxyl group; an alkoxycarbonyl group having 2 to 10 carbon atoms (for example, methoxycarbonyl group, ethoxycarbonyl group and the like); cyano group; a substituted or unsubstituted carbamoyl group having 1 to 10 carbon atoms (for example, carbamoyl group, methylcarbamoyl group, morpholinocarbamoyl group and the like); a substituted or unsubstituted amino group (for example, an amino group substituted with an alkyl group having 1 to 20 carbon atoms such as methylamino group, dimethylamino group, ethylmethylamino group, acetylamino group, disulfonylethylamino group and N-ethyl-N'-sulfoethylamino group); sulfo group; oxo group and the like. When these substituents form a salt, the compounds represented by the formula (1) may be present in the form of a salt.

As compounds that form a stable complex with a metal ion of an active center of an enzyme, compounds represented by formulas (6), (7) and (8) can also be used.

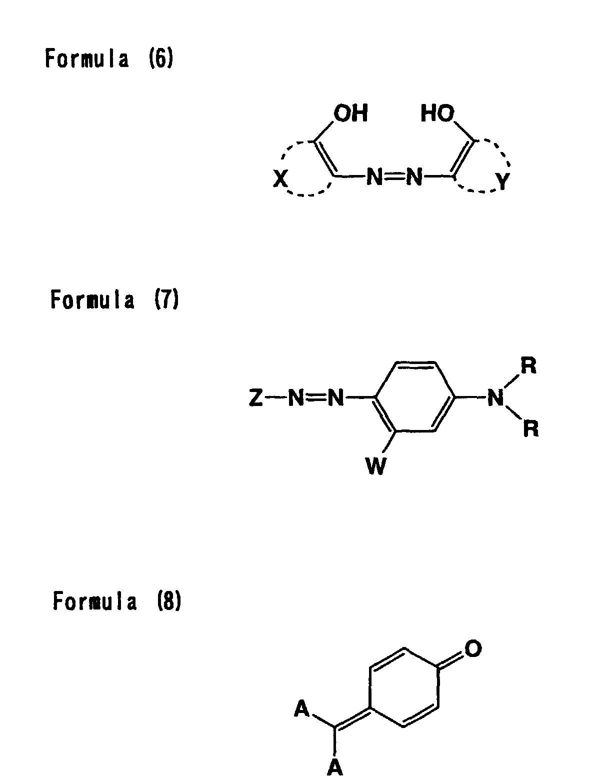

In the formulas (6) to (8), X and Y independently represent a group of atoms required for forming an aryl group (for example, phenyl group, naphthyl group) or a heteroaryl group (for example, pyridinyl group, quinolinyl group). The aryl group or the heteroaryl group may have one or more substituents other than each of the one hydroxyl group shown in the formula (6). X and Y may be the same or different, and an aryl group may preferably be used as the groups. An aryl group substituted with one or more water-soluble groups may most preferably be used.

Z represents a substituted or unsubstituted nitrogen-containing heteroaryl group, and preferably pyridyl group, benzothiazolyl group, thiazolyl group, quinolyl group and the like may be used. More preferably, a 2-pyridyl group may be used which is substituted with one or more halogen atoms that may be the same or different. W represents amino group, hydroxyl group, or carboxyl group. When matrix metalloproteinase is the object enzyme of the measurement, and the zinc ion in the enzyme is detected, W may preferably be carboxyl group or hydroxyl group, more preferably hydroxyl group.

Specific examples of the compounds represented by each formula are shown below. However, compounds used for the preparation of the thin membrane of the present invention are not limited to these examples. The compounds represented by the formula (1) are novel. Methods for preparation of their typical compounds are specifically explained in detail in the examples of the specification, and accordingly, those skilled in the art can easily prepare any compounds falling within formula (1).

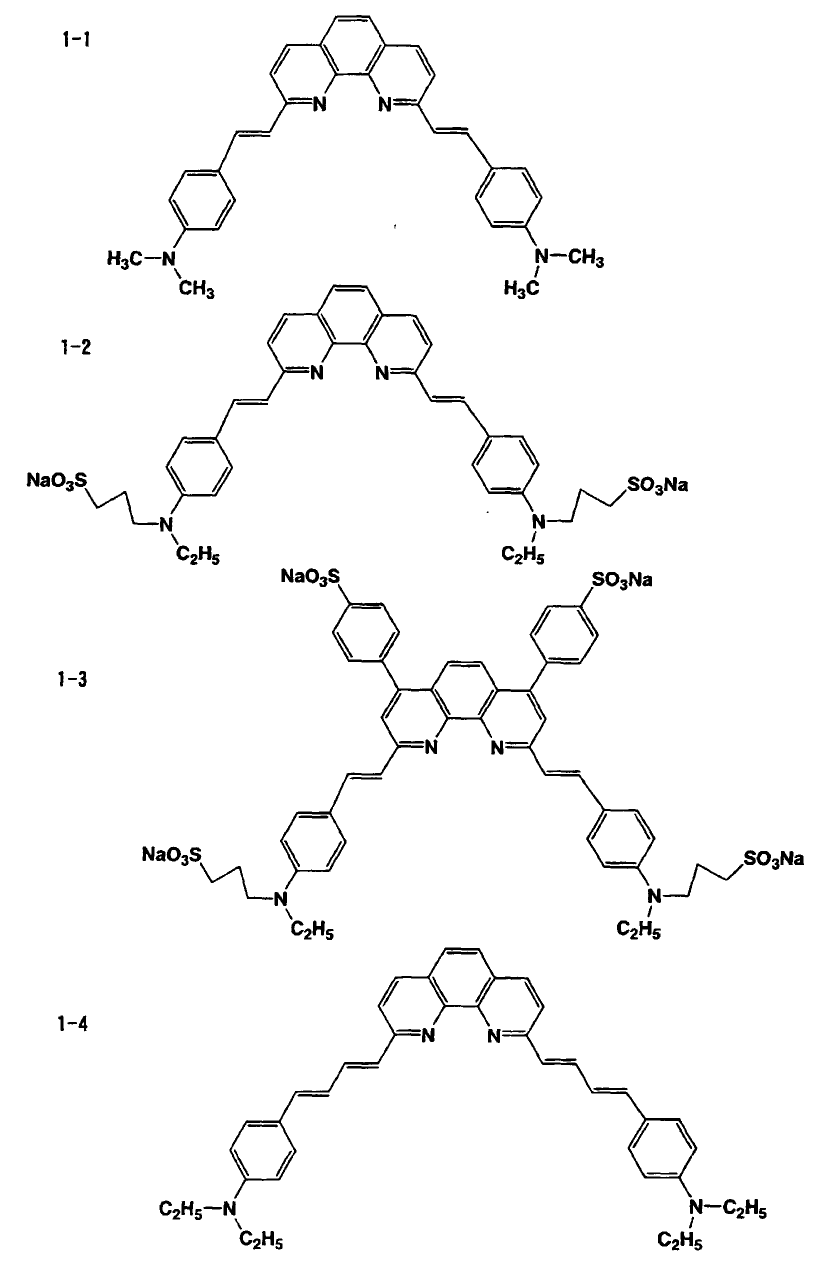

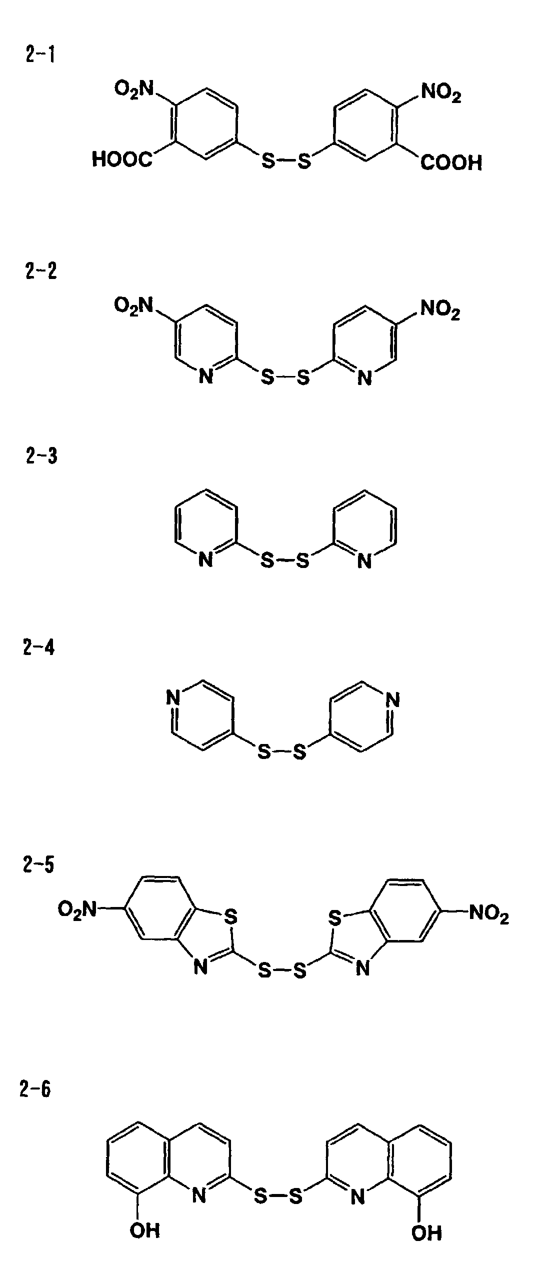

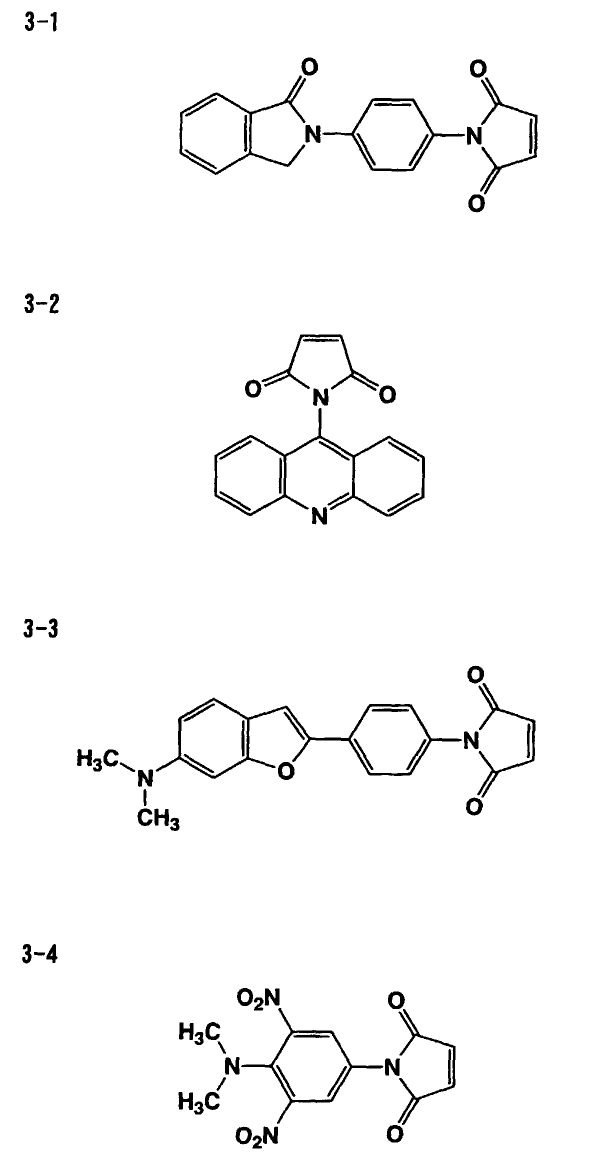

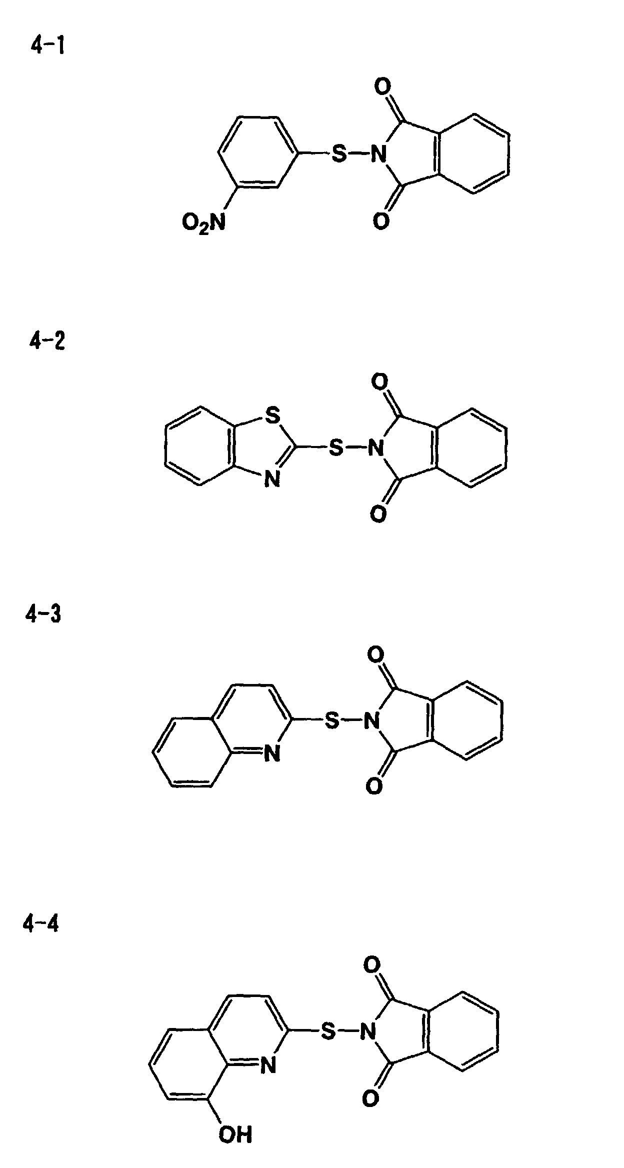

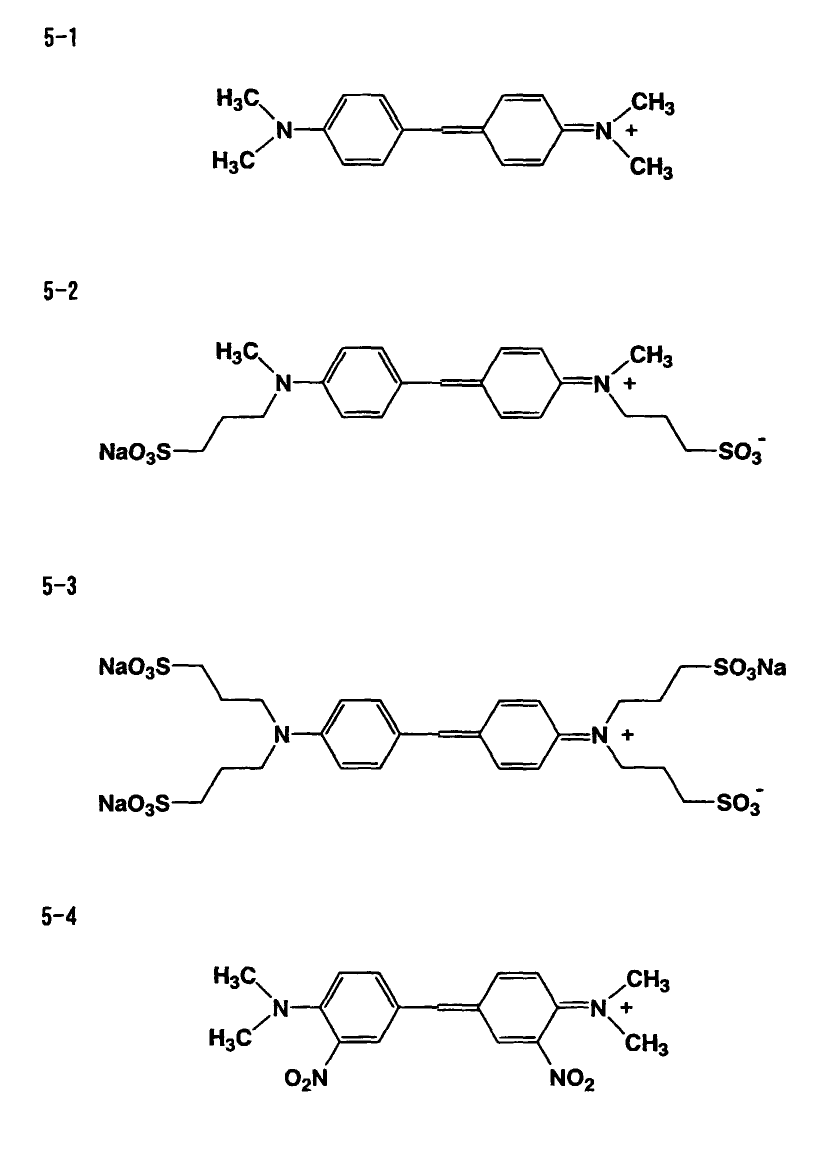

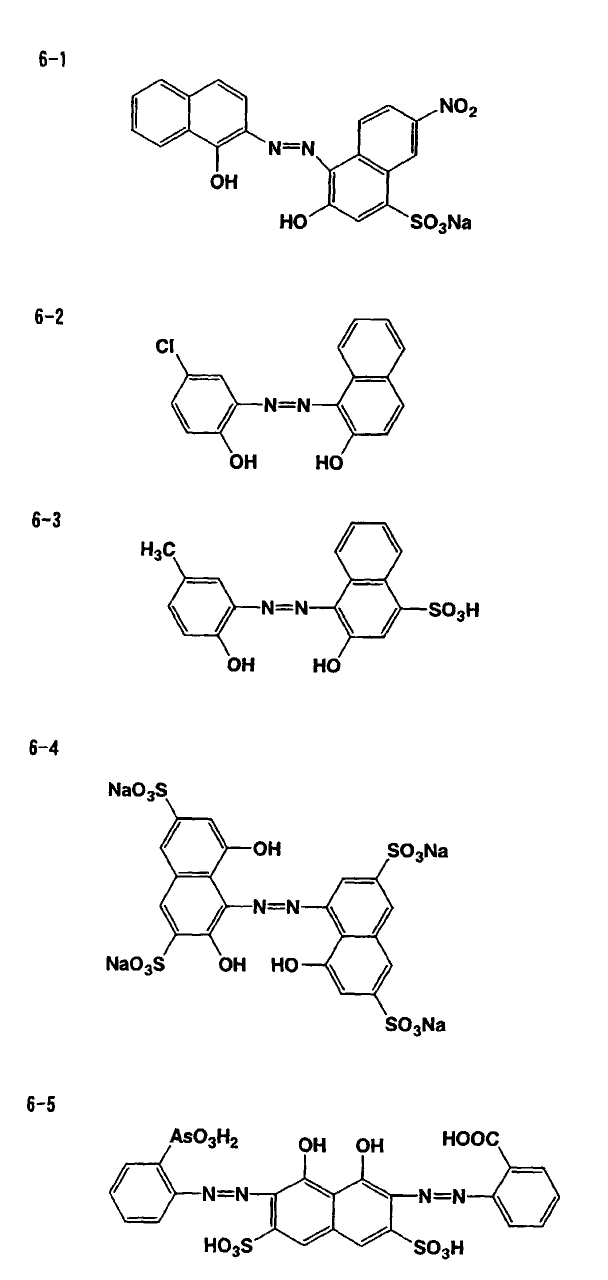

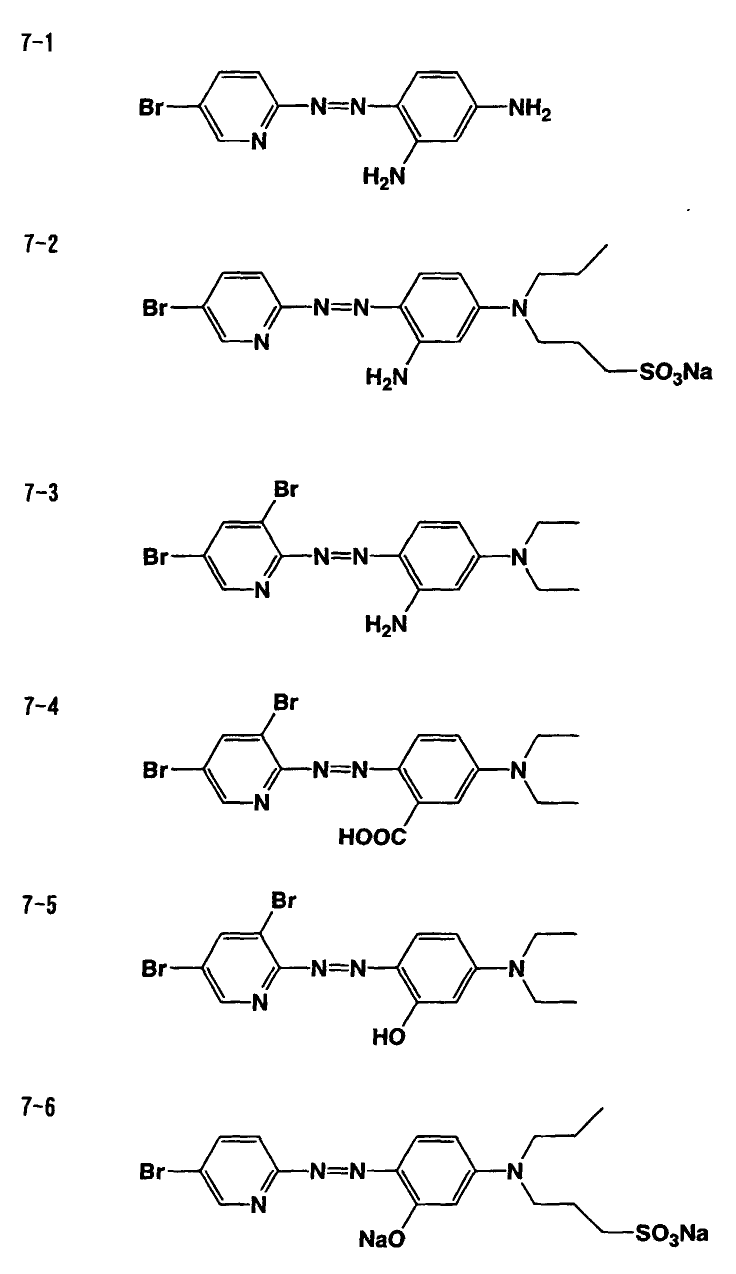

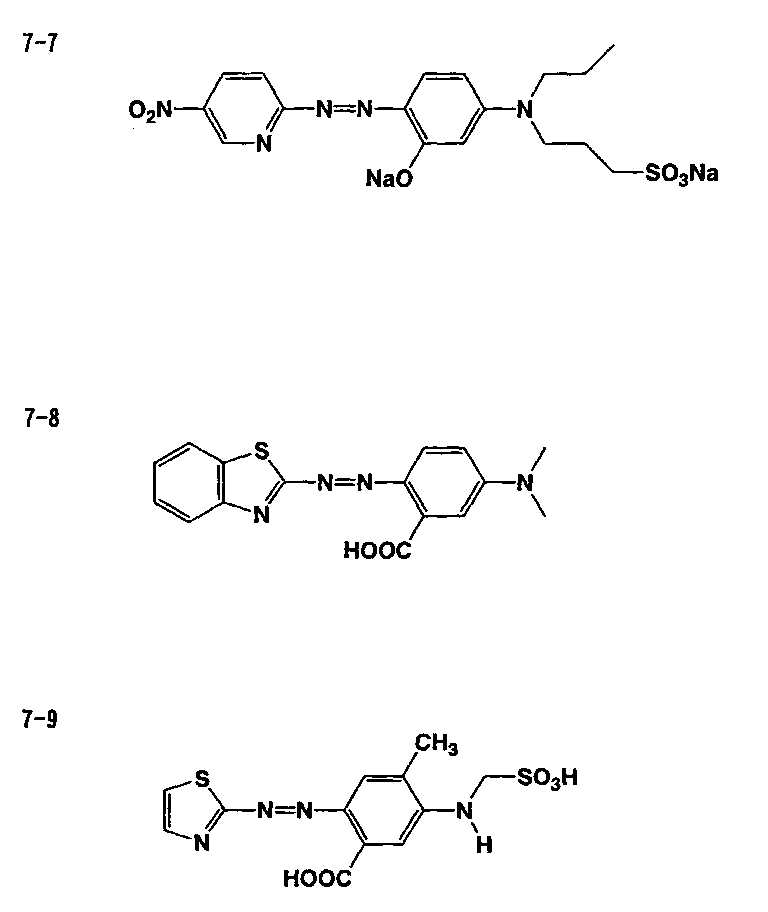

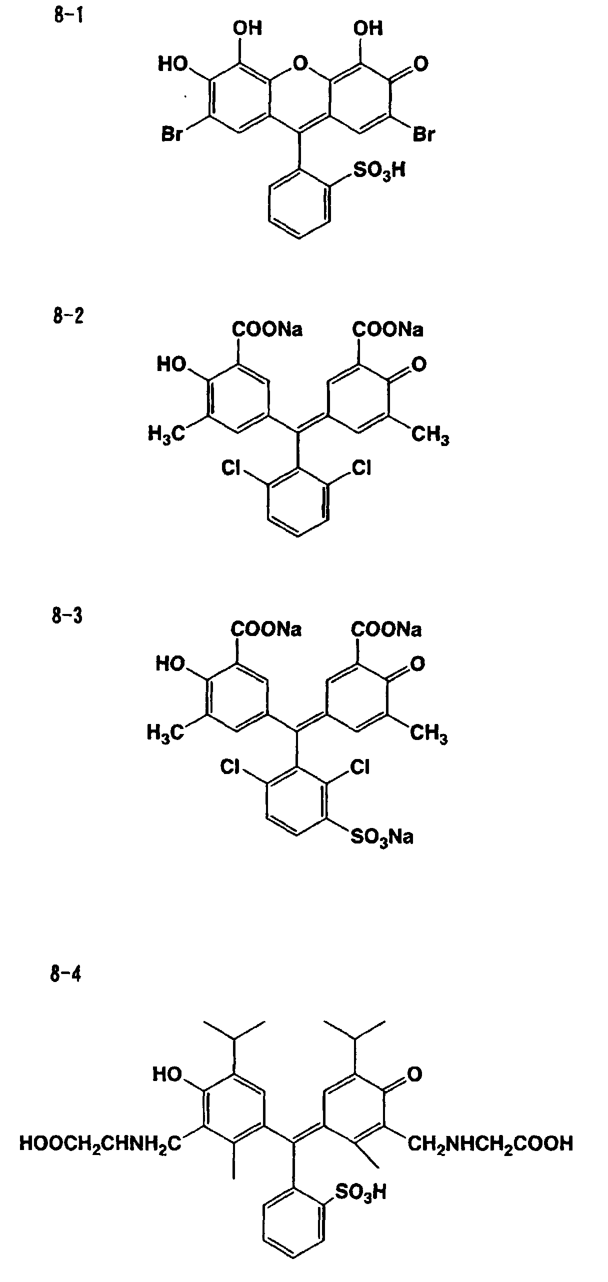

The organic compound need to be present in the thin membrane, however, existing state thereof is not particularly limited. Such state may be preferred in which the compound is added as a solution or the compound is dispersed in a hydrophilic substrate. The amount of the organic compound contained in the thin membrane can be appropriately chosen depending on the type of a hydrophilic polymer, the degree of a change in visible absorption, the detection means for the change, the type of a sample and the like. For example, when gelatin or the like is used as the hydrophilic polymer, the compound may preferably be added in an amount of from about from 1×10⁻⁵ mol/m² to 1.0 mol/m².

As the hydrophilic polymer for forming a thin membrane used in the methods of the present invention, any hydrophilic polymers may be used which are dissolved in water or swelled by absorbing water. As natural hydrophilic polymers, for example, proteins and substances derived from proteins such as gelatin, collagen, casein, fibronectin, laminin and elastin; polysaccharides and substances derived from polysaccharides such as cellulose, starch, agarose, carrageenan, dextran, dextrin, chitin, chitosan, pectin, and mannnan may be used. As synthetic hydrophilic polymer, for example, polyvinyl alcohol (Poval), polyacrylamide, polyacrylic acid, polyvinylpyrrolidone, polyethylene glycol, polystyrenesulfonic acid, polyallylamine and the like may be used. Gels derived from these substances may also be used. However, the hydrophilic polymers are not limited to these examples. When gelatin is used as the hydrophilic polymer, for example, alkali extracted bovine bone gelatin, alkali extracted swine cutis gelatin, acid extracted bovine bone gelatin, phthalation-treated bovine bone gelatin, acid extracted swine cutis gelatin and the like may be used.

In order to prevent swelling of the thin membrane, one or more inorganic or organic hardening agents may be formulated in the thin membrane. Such hardening agents may be appropriately chosen from those available for accelerating curing of thin membranes. However, the agents need to be chosen so as not to affect enzymatic activity to be measured. For example, active halogen compounds (2,4-dichlor-6-hydroxy-1,3,5-triazine and its sodium salt and the like), active vinyl compounds (1,3-bisvinylsulfonyl-2-propanol, 1,2-bis(vinylsulfonylacetamido)ethane, bis(vinylsulfonylmethyl) ether, vinyl polymers having vinylsulfonyl groups on the side chains and the like) may be used, and 1,2-bis(vinylsulfonylacetamido)ethane may preferably be used.

By using an enzyme inhibitor such as a protease inhibitor, identification of an enzyme that relates to the inhibitor or determination of properties of an enzyme such as proteases may be facilitated, and thereby accuracy of pathological examinations may be enhanced. When a protease is a measurement object in the method of pathological examination according to the present invention, examples of the protease inhibitors include, for example, tissue inhibitor of metaproteinase 1 (TIMP1), tissue inhibitor of metaproteinase 2 (TIMP2), large inhibitor of metalloproteinase (LIMP), chicken inhibitor of metalloproteinase (ChIMP), opostatin, platelet factor IV (PF-4), α₂ macroglobulin, EDTA, 1,10-phenanthroline, BB94, minocycline, matristatin, SC-44463, dithiothreitol (DTT) and the like.

For example, a method may be employed wherein one of two or more substantially continuous slices of a biosample is brought into contact with the aforementioned thin membrane not containing an enzyme inhibitor; and the remaining slice is brought into contact with the aforementioned thin membrane containing a protease inhibitor; and then traces formed on each of the thin membranes are compared. In addition, for example, a method may also be employed wherein a single thin membrane is prepared which comprises a first layer not containing an enzyme inhibitor and a second layer containing an enzyme inhibitor; a sample containing an enzyme is brought into contact with the thin membrane; and then traces formed on each of the layers are compared. Two or more enzyme inhibitors may be used in combination for formulation of a single hydrophilic polymer thin membrane, or a hydrophilic polymer thin membrane comprising layers each containing two or more enzyme inhibitors may also be used.

A thin membrane containing a substance degradable by an enzyme as a measuring object (an enzyme substrate) may be manufactured, and then one of two or more substantially continuous slices of a biosample may be brought into contact with the thin membrane containing the enzyme substrate, and the remaining slice(s) may be brought into contact with the aforementioned thin membrane not containing the enzyme substrate. Traces of digestion are formed by the action of the enzyme on the thin membrane containing the enzyme substrate, whereas traces showing change in visible absorption are formed on the thin membrane of the present invention. Therefore, the presence of the enzyme can be reliably proved by comparing the results to each other. The method using an enzyme substrate may be appropriately combined with the aforementioned method using an enzyme inhibitor. The measurement can also be performed by using a thin membrane obtained by laminating a layer containing an enzyme substrate and a layer not containing the enzyme substrate.

When a protease is measured as the enzyme, a macromolecular compound degradable by the protease can be used as an enzyme substrate (a protease substrate). The protease substrate is not particularly limited. For example, collagen, gelatin, proteoglycan, fibronectin, laminin, elastin, casein and the like may be used. Preferably, collagen, gelatin, fibronectin, elastin or casein may be used, and gelatin, fibronectin and casein are more preferably used. When gelatin is used, a type of gelatin is not particularly limited. For example, alkali extracted bovine bone gelatin, alkali extracted swine cutis gelatin, acid extracted bovine bone gelatin, phthalation-treated bovine bone gelatin, acid extracted swine cutis gelatin and the like can be used. As the protease substrate, one of the aforementioned substances may be used alone, or two or more of the substrates may be used in combination.

By using two or more different protease substrates in combination, a type of a protease contained in a biosample can sometimes be accurately identified. For example, a method may be employed wherein one of two or more substantially continuous slices of a biosample are brought into contact with the thin membrane of the present invention, and traces formed on the thin membrane are detected; and the other two or more slices are brought into contact with two or more thin membranes each containing a different protease substrate, and traces of digestion are detected; and then the results obtained are compared.

The samples used for the methods of the present invention are not particularly limited. For example, biosamples isolated or collected from mammals including human or biosamples excreted from mammals including humans may be used. For example, tissues or tissue exudate and the like may be used. More specifically, cancer tissues from solid tumor tissues of lung cancer, stomach cancer, esophageal cancer, breast cancer, brain tumor and the like which are isolated or collected by surgical operation or histological examination; synovial fluid and bone tissue of rheumatoid arthritis; destructive morbid tissues such as periodontal ligament or bone tissue and exudate of alveolar pyorrhea; gingival crevicular exudate of periodontal diseases and the like may be used. Cultured cells and tissues may also be used.

When a tissue is used as a sample, for example, a slice having a thickness of 1 to 10 µm, preferably 5 µm may be prepared from a sample rapidly frozen in liquid nitrogen by using an apparatus for preparing frozen sections, and then the slice may be applied to a thin membrane to bring the sample contact with the thin membrane. When synovial fluid collected from a patient of rheumatoid arthritis is used as a sample, about 5 to 50 µl, preferably about 20 µl of synovial fluid can be dropped onto the thin membrane. When gingival crevicular fluid of periodontal disease is used as a sample, a piece of filter paper may be inserted into gingival crevice to collect about 5 to 10 µl of gingival crevicular fluid, and the filter paper may be applied to a thin membrane. After the collection of gingival crevicular fluid, the gingival crevicular fluid may be optionally extracted from the filter paper using distilled water or a suitable buffer (for example, 50 mM Tris-HCl, pH 7.5, 10 mM CaCl₂, 0.2 M NaCl), and the extract may be dropped onto a thin membrane.

The thin membrane is preferably formed on a flat surface of a support. Examples of transparent or translucent supports include, for example, glass and transparent or translucent plastic films made of polyethylene terephthalate, polycarbonate, polyimide, nylon, cellulose, triacetate and the like. As glass, it is preferred to use a microscope slide, and as a plastic film, it is preferred to use a polyethylene terephthalate film. As opaque supports, for example, paper, synthetic paper, paper laminated with synthetic resin (for example, polyethylene, polypropylene, polystyrene, polyethylene naphthalate or the like), metal plates (for example, plates of aluminium, aluminium alloy, zinc, iron, copper or the like), paper or plastic films laminated or deposited with the aforementioned metals and the like may be used.

Material and shape of the support are not particularly limited so long as a uniform thin membrane can be manufactured and change in absorption of visible light is detectable. Therefore, the supports are not limited to those specifically exemplified above. The support may have a porous internal structure. Examples include ceramic plates, polyurethane foams, polysulfone having micro voids, polycarbonate provided with fine holes and the like. The support may be colored.

The thickness of the support is not particularly limited. As to glass supports, those having a usual thickness of a microscope slide (for example, approximately 2 to 3 mm) may be preferred. As to polyethylene terephthalate films, those having a thickness of about 100 to 250 µm, more preferably about 150-200 µm, and most preferably about 175 µm may be used. The thin membrane on the support can be formed as monolayer or multilayer. The thin membrane should be prepared so as to have a surface as uniform as possible. For example, the thin membrane may preferably be formed so as to have a thickness of about 1 to 10 µm, more preferably about 4-6 µm after drying.

For the preparation of the thin membrane, for example, given amounts of the organic compound and the hydrophiic polymer may be dispersed in water or an organic solvent such as methylene chloride, acetone, methanol and ethanol, or a mixed solvent thereof, and then the resulting dispersion may be applied to a surface of a support and dried. As methods for coating, dip coating method, roller coating method, curtain coating method, extrusion coating method and the like can be employed. However, methods for preparing the thin membrane are not limited to these examples, and methods conventionally used for the preparation of thin membranes in the field of photographic films, for example, may be appropriately employed.

When a thin membrane is formed on a support, an undercoat layer may be provided between the thin membrane and the support in order to improve the adhesion of the thin membrane and the support. For example, the undercoat layer may be formed by using a polymer or a copolymer obtained by polymerization of one or more monomers selected from vinyl chloride, vinylidene chloride, butadiene, methacrylic acid, acrylic acid, itaconic acid, maleic anhydride and the like, or a polymer such as polyethyleneimine, epoxy resin, grafted gelatin, nitrocellulose and the like. When a polyester support is used, adhesion between the support and the thin membrane may sometimes be improved by subjecting the surface of the support to corona discharge treatment, ultraviolet irradiation or glow discharge treatment instead of providing the undercoat layer.

The wording "a thin membrane formed on a surface of a support" and equivalents thereof used in the present specification should not be construed to exclude those with one or more undercoat layers and/or a surface treatment of the support. Means to improve the adhesion of the thin membrane and the support are not limited to those mentioned above, and for example, those conventionally used in the field of photographic film and other fields can be appropriately used. When the thin membrane is formed by laminating a plurality of layers as described above, an intermediate layer can further be provided between two laminated layers. The term "laminated" used in the present specification should not be construed so as to limit laminated two layers to those in direct contact. Means for appropriately providing such intermediate layers are generally used, for example, in the field of photographic film and the like.

For the preparation of the thin membrane, other components such as dyes, pigments, antiseptics, stabilizers and the like may optionally be added in addition to the components mentioned above. These components are not particularly limited, and may be appropriately chosen and employed so long as they do not substantially inhibit the reaction of an enzyme to be measured and the aforementioned organic compound. For example, as the dyes, those disclosed in the Japanese Patent Unexamined Publication (Kokai) No. (Hei) 6-102624/1994 (dyes specifically represented by the formula I-1 on page 9 to the formula 63 on page 47) can be used. As methods for adding dyes, those disclosed in the Japanese Patent Unexamined Publication (Kokai) No. (Hei) 5-313307/1993 (methods specifically explained from paragraph [0037] on page 11 to paragraph [0044] on page 12) can be used.

Modes for performing the methods of the present invention are not particularly limited. For example, a sample containing an enzyme such as a protease is brought into contact with a thin membrane by, for example, applying a tissue slice to the thin membrane or dropping a liquid sample onto the thin membrane, and then incubation is carried out preferably in a humidified box at 37°C for a period of, for example, about 1 to 24 hours, preferably about 2 to 12 hours, more preferably about 3 to 6 hours for tissue slices, or about 0.5 to 12 hours, preferably about 1 to 6 hours, more preferably about 1 to 3 hours for liquid samples.

When an enzyme such as a protease is contained in the sample, a reaction between the aforementioned organic compound in the thin membrane and the enzyme occurs, and the traces with change in visible absorption are formed on the thin membrane. Further, when the thin membrane is formed by using as the hydrophilic polymer a macromolecular compound as an enzyme substrate (for example, when the thin membrane is formed by using a protease substrate such as gelatin as the hydrophilic polymer for protease measurement), traces of digestion formed by the action of the enzyme are sometimes detectable simultaneously.

According to another embodiment of the method of the present invention, the presence of an enzyme such as a protease derived from each individual cells in a tissue can be accurately determined by preparing continuous frozen sections from a cancer tissue or the like, and then preparing a conventional histopathological preparation of one of the two substantially continuous slices such as a hematoxylin/eosin staining slice, and treating the other slice according to the method of the present invention, followed by comparing and distinguishing the results of observation to each other. Use of the method of the present invention for measurement of a protease enables accurate determination of malignancy (infiltrative and metastatic activity) of individual cancer cells present in tissues, and definite diagnosis of the prognosis of the cancer. By quantitative measurement of protease in a sample of synovial fluid from rheumatoid arthritis, gingival crevicular fluid and the like, pathological conditions and the degree of progress of diseases can be accurately determined. For the quantitative measurement of a protease in a sample, it is desirable to prepare a calibration curve using a standard solutions prepared beforehand.

### Examples

The present invention will be explained more specifically with reference to the following examples. However, the scope of the present invention is not limited to these examples. The compound numbers used in the examples correspond to the numbers of the preferred compounds mentioned above.

### Example 1: Synthesis of Compound 1-1

2,9-Dimethyl-1,10-phenanthroline (4.34 g) and 4-dimethylaminobenzaldehyde (6.0 g) were dissolved in t-butanol (50 ml), and the solution was added with potassium t-butoxide (4.6 g) and then allowed to react at 100°C for 20 minutes. The crystals deposited in the reaction system were collected by filtration and recrystallized from a mixed solvent of chloroform and methanol to obtain Compound 1-1 (5.2 g, yield: 55%). H-NMR: δ 8.15 (2H, d), 7.89 (2H, d), 7.70-7.50 (10H, m), 6.77 (4H, d), 3.05 (12H, s)

### Example 2: Synthesis of Compound 1-2

2,9-Dimethyl-1,10-phenanthroline (2.17 g) and sodium salt of 4-(N-ethyl-N-sulfopropyl)aminobenzaldehyde (7.6 g) were dissolved in t-butanol (50 ml), and the solution was added with potassium t-butoxide (2.3 g) and then allowed to react at 100°C for 3 hours. The crystals deposited in the reaction system was collected by filtration and washed with hot methanol to separate and remove tar. The methanol solution was added with sodium acetate to deposit Compounds 1-2 (3.6 g, yield: 48%).

The structure was verified by the peak at 735 (M-Na) in a mass spectrum.

### Example 3: Synthesis of Compound 1-3

2,9-Dimethyl-4,7-diphenyl-1,10-phenanthrolinedisulfonic acid disodium salt (5.64 g) and sodium salt of 4-(N-ethyl-N-sulfopropyl)aminobenzaldehyde (7.6 g) were dissolved in t-butanol (50 ml), and the solution was added with potassium t-butoxide (2.3 g) and then allowed to react at 100°C for 3 hours. The crystals deposited in the reaction system were collected by filtration and recrystallized from a mixed solvent of water and methanol to obtain Compound 1-3 (7.8 g, yield: 70%).

The structure was verified by the peak at 1091 (M-Na) in a mass spectrum.

### Example 4: Synthesis of Compound 1-4

2,9-Dimethyl-1,10-phenanthroline (2.17 g) and 4-dimethylaminocinnamaldehyde (3.5 g) were dissolved in t-butanol (25 ml), and the solution was added with potassium t-butoxide (2.3 g) and then allowed to react at 100°C for 1 hour. The crystals deposited in the reaction system were collected by filtration and recrystallized from a mixed solvent of chloroform and methanol to obtain Compound 1-4 (2.0 g, yield: 35%).

The structure was confirmed by the peak at 579 (M+H) in mass spectrum.

### Example 5: Preparation of thin membrane for measurement of protease

### [Monolayer thin membrane: Preparation of Sample 101]

Acid extracted swine cutis gelatin (15 g) was dissolved in pure water (122 g), and the solution was added with Compound 1-2 (1.2 ml, 5% aqueous solution), and 1,2-bis(vinylsulfonylacetamido)ethane (4%, 0.6 ml) as a hardening agent. The solution was uniformly applied to a microscope slide by a wire bar coater so as to obtain a membrane of dry thickness of about 7 µm, and then the membrane was dried to obtain a thin membrane. The thin membrane was stored at room temperature before use.

### [Monolayer thin membrane: Preparation of Samples 102 to 146]

The enzyme-reactive organic compound, hydrophilic polymer, hardening agent, additive, and support were changed or added as shown in Tables 1 and 2 to prepare Samples 102 to 146 in the same manner as the preparation of Sample 101. For the coating of solutions, a coating aid was used as required.

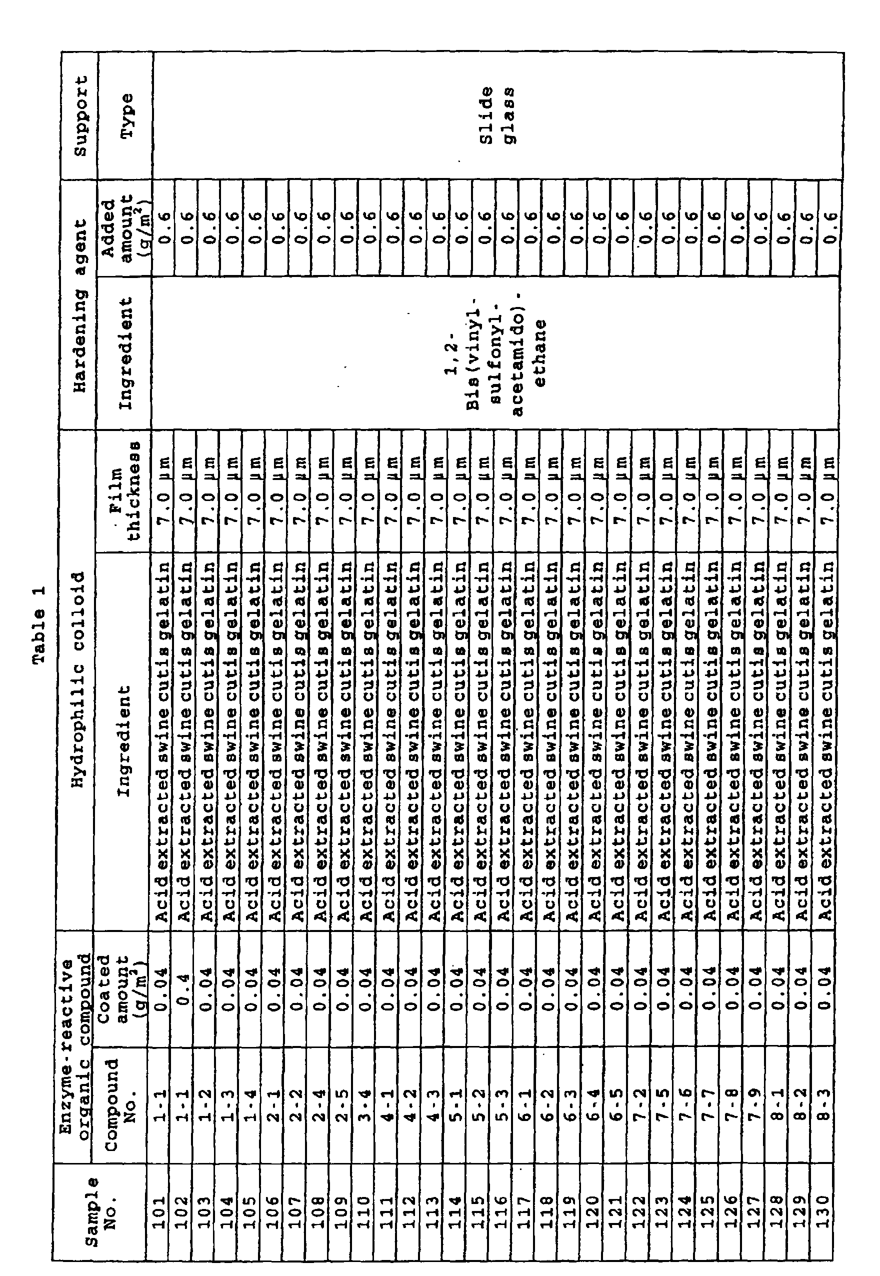

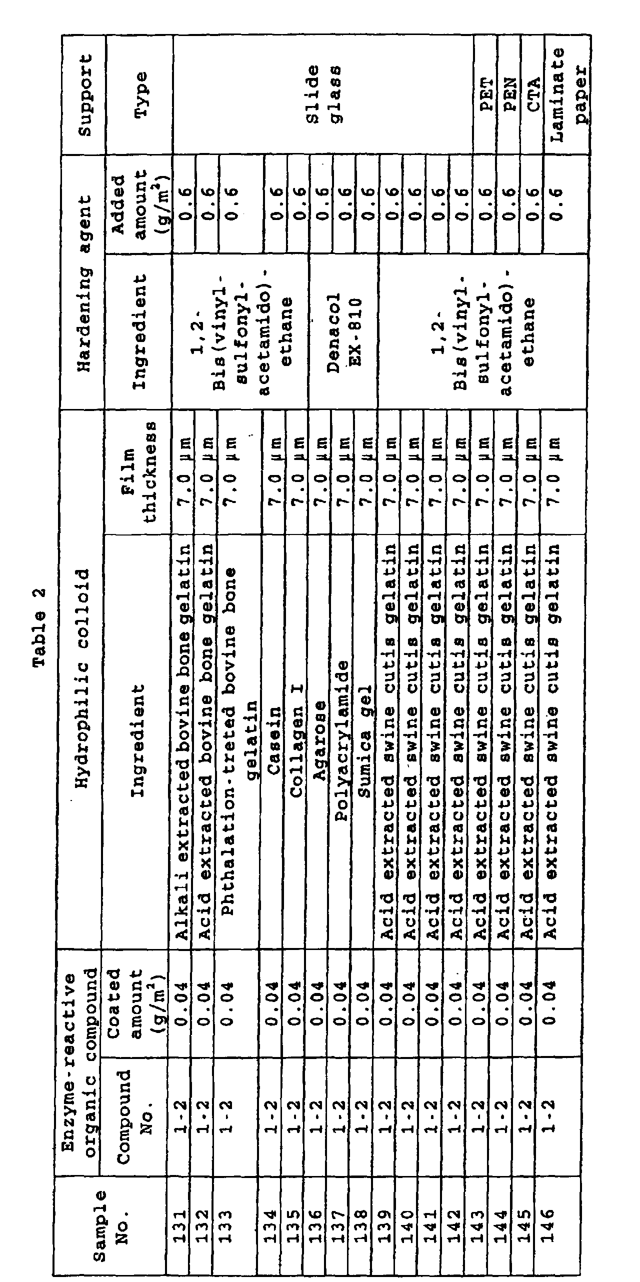

### Example 6: Measurement of protease activity using thin membrane

### [Measurement for liquid sample: Measurement Method A]

As protease liquid samples, solutions each containing matrix metalloproteinase (MMP)-1, MMP-2 or MMP-9 (Yagai Co., Ltd.) at a concentration of from 2 pg/ml to 200 ng/ml were used. As biosamples, gingiva and gingival crevicular fluid (GCF) collected from patients with a periodontal disease and culture supernatants of pathogenic causal bacteria of periodontal diseases (P. Gingivalis #381 strain; A. actinomycetemcomitans Y4 strain; and P. intermedia ATCC 25611 strain) were used. About 10 µl of liquid samples were dropped onto each of the thin membranes obtained in Example 5. The thin membranes were placed in a humidified box and incubated at 37°C for 4 to 16 hours. For the evaluation of the activity, changes in absorption at particular wavelengths each for reacted and unreacted sites were determined for every samples by a microspectrophotometer. The results are shown in Table 3.

**Table 3**

| Sample No. | Measured wavelength (nm) | Reflected optical density | | Sample No. | Measured wavelength (nm) | Reflected optical density | |
|---|---|---|---|---|---|---|---|
| | | Unreacted site | Reacted site | | | Unreacted site | Reacted site |
| 101 | 510 | 0.06 | 2.05 | 131 | 510 | 0.05 | 2.46 |
| 102 | 510 | 0.31 | 4.55 | 132 | 510 | 0.05 | 2.47 |
| 103 | 510 | 0.05 | 2.44 | 133 | 510 | 0.05 | 2.52 |
| 104 | 510 | 0.05 | 2.13 | 134 | 510 | 0.05 | 2.37 |
| 105 | 600 | 0.75 | 2.98 | 135 | 510 | 0.05 | 2.46 |
| 106 | 440 | 0.05 | 1.12 | 136 | 510 | 0.05 | 2.41 |
| 107 | 420 | 0.06 | 1.09 | 137 | 510 | 0.05 | 2.44 |
| 108 | 400 | 0.04 | 1.32 | 138 | 510 | 0.05 | 2.45 |
| 109 | 440 | 0.06 | 1.45 | 139 | 510 | 0.05 | 2.13 |
| 110 | 440 | 0.11 | 1.12 | 140 | 510 | 0.05 | 2.47 |
| 111 | 400 | 0.03 | 0.98 | 141 | 510 | 0.05 | 2.51 |
| 112 | 400 | 0.07 | 1.05 | 142 | 510 | 0.05 | 2.48 |
| 113 | 400 | 0.04 | 0.95 | 143 | 510 | 0.05 | 2.35 |
| 114 | 606 | 1.38 | 0.03 | 144 | 510 | 0.05 | 2.11 |
| 115 | 606 | 1.45 | 0.02 | 145 | 510 | 0.05 | 2.35 |
| 116 | 606 | 1.48 | 0.04 | 146 | 510 | 0.05 | 2.33 |
| 117 | 620 | 0.25 | 3.53 | | | | |
| 118 | 575 | 0.35 | 3.35 | | | | |
| 119 | 690 | 0.21 | 2.99 | | | | |
| 120 | 550 | 0.15 | 3.23 | | | | |
| 121 | 600 | 0.22 | 2.57 | | | | |
| 122 | 550 | 0.41 | 3.53 | | | | |
| 123 | 590 | 0.06 | 3.48 | | | | |
| 124 | 552 | 0.35 | 3.21 | | | | |
| 125 | 566 | 0.23 | 3.58 | | | | |
| 126 | 640 | 0.38 | 3.53 | | | | |
| 127 | 550 | 0.28 | 3.16 | | | | |
| 128 | 550 | 0.55 | 2.05 | | | | |
| 129 | 600 | 0.34 | 2.64 | | | | |
| 130 | 600 | 0.23 | 1.79 | | | | |

Visible colored portions and decolorized portions appeared on every sample. The optical density of these portions increased compared with surrounding portions as shown in Table 3. As to MMP-1, MMP-2 and MMP-9, protease activities were observed at a concentration of 200 ng/ml after 4 hours, at 20 ng/ml after 8 hours, and at a concentration ranging from 20 to 200 pg/ml after 16 hours. Most of the gingival crevicular fluids required 8 to 16 hours until protease activity was observed, and it was suggested that the amount of protease was in a range of from 2 pg/ml to 20 ng/ml.

### [Measurement for frozen slice sample of a living body: Measurement Method B]

### 〈Measurement of protease activity for gingiva, gingival crevicular fluid and periodontal disease tissue〉

As biosamples, gingiva, gingival crevicular fluid and periodontal disease tissue samples were applied to each of the thin membrane samples 101 to 146 as frozen slices of about 5 µm. The thin membranes were placed in a humidified box and incubated at 37°C for 4 to 16 hours. For evaluation of the activity, changes in absorption at particular wavelengths each for microportions of the reacted and unreacted sites were determined for every sample by a microspectrophotometer. The results are shown in Table 4. In the present method where the frozen slices of biosamples were directly placed, colored sites and decolorized sites formed by a protease were also observed on every thin membrane.

**Table 4**

| Sample No. | Measured wavelength (nm) | Reflected optical density | | Sample No. | Measured wavelength (nm) | Reflected optical density | |
|---|---|---|---|---|---|---|---|
| | | Unreacted site | Reacted site | | | Unreacted site | Reacted site |
| 101 | 510 | 0.05 | 2.15 | 131 | 510 | 0.05 | 2.44 |
| 102 | 510 | 0.22 | 4.35 | 132 | 510 | 0.05 | 2.43 |
| 103 | 510 | 0.05 | 2.44 | 133 | 510 | 0.05 | 2.52 |
| 104 | 510 | 0.04 | 2.34 | 134 | 510 | 0.05 | 2.53 |
| 105 | 600 | 0.73 | 3.05 | 135 | 510 | 0.05 | 2.24 |
| 106 | 440 | 0.04 | 1.15 | 136 | 510 | 0.05 | 2.28 |
| 107 | 420 | 0.05 | 1.12 | 137 | 510 | 0.05 | 2.21 |
| 108 | 400 | 0.04 | 1.36 | 138 | 510 | 0.05 | 2.35 |
| 109 | 440 | 0.06 | 1.51 | 139 | 510 | 0.05 | 2.41 |
| 110 | 440 | 0.1 | 1.15 | 140 | 510 | 0.05 | 2.43 |
| 111 | 400 | 0.02 | 1.12 | 141 | 510 | 0.05 | 2.44 |
| 112 | 400 | 0.02 | 1.21 | 142 | 510 | 0.05 | 2.54 |
| 113 | 400 | 0.05 | 1.11 | 143 | 510 | 0.05 | 2.33 |
| 114 | 606 | 1.76 | 0.01 | 144 | 510 | 0.05 | 2.31 |
| 115 | 606 | 1.98 | 0.03 | 145 | 510 | 0.05 | 2.43 |
| 116 | 606 | 2.04 | 0.02 | 146 | 510 | 0.05 | 2.43 |
| 117 | 620 | 0.23 | 3.55 | | | | |
| 118 | 575 | 0.22 | 3.43 | | | | |
| 119 | 690 | 0.25 | 3.01 | | | | |
| 120 | 550 | 0.11 | 3.25 | | | | |
| 121 | 600 | 0.21 | 2.54 | | | | |
| 122 | 550 | 0.46 | 3.64 | | | | |
| 123 | 590 | 0.11 | 3.24 | | | | |
| 124 | 552 | 0.21 | 3.24 | | | | |
| 125 | 566 | 0.18 | 3.35 | | | | |
| 126 | 640 | 0.34 | 3.64 | | | | |
| 127 | 550 | 0.25 | 3.13 | | | | |
| 128 | 550 | 0.61 | 2.11 | | | | |
| 129 | 600 | 0.35 | 1.87 | | | | |
| 130 | 600 | 0.24 | 1.88 | | | | |

### 〈Measurement of protease in cancer tissues〉

As test tissue samples, surgical specimens of glossa epidermoid cancer, lung cancer and esophageal cancer were cut into pieces of about 0.5 cm thickness × 2 cm width, and rapidly frozen in liquid nitrogen and then stored at -80°C. Continuous slices having a thickness of 5 µm were prepared from these samples using a frozen section preparing apparatus, and one of the slices was affixed to a microscope slide and dried. Then, the slice was fixed with 10% formalin for 5 minutes, and stained with hematoxylin/eosin in a conventional manner. Other continuous slices were affixed on the thin membranes produced in Example 5, and the membranes were placed in a humidified box and incubate at 37°C for 3 to 6 hours.

After the incubation, the thin membranes gave colored or decolorized traces formed by a protease. In every cancer tissue, colored or decolorized traces formed by a protease were observed in individual cancer cells forming cancer alveolus lesions, and the cells located in peripheries of cancer alveolus lesions gave particularly distinct colored or decolorized traces formed by a protease. Weak colored or decolorized traces were also observed in normal platycytes, and correlating to the progress of atypical epithelium growth, stronger reaction by a protease was recognized. Portions diagnosed as cancer cells by hematoxylin/eosin staining gave colored or decolorized traces formed by a protease at exactly the same locations on the thin membranes of the present invention. In addition, sites with newly developed activity and already cornified sites were also clearly recognized.

### 〈Measurement of protease activity in buccal cavity maxillary gingiva cancer〉

Cancer cells in a sample were those of low-differentiated epidermoid cancer which formed an alveolus lesion structure, and they destroyed bone tissues and showed strong infiltration. Portions of thin membrane, corresponding to the cancer cells in the cancer alveolus lesions, gave colored or decolorized traces formed by a protease, and in particular, portions of the thin membrane corresponding to the cells located in peripheries of the cancer alveolus lesions gave distinct colored or decolorized traces formed by a protease. At portions corresponding to focal inflammatory cell infiltration sites, remarkable granular colored or decolorized traces were formed by a protease. When the cancer alveolus lesions were observed in magnification, distinct colored or decolorized traces formed by a protease were recognized in the cells of growing sites that located at peripheries of the cancer alveolus lesions, and granular colored or decolorized traces formed by a protease were observed also at fibroblasts in interstitium adjacent to the cancer alveolus lesions.

### 〈Measurement of protease activity in lingual cancer〉

Cancer cells in a sample were those of undifferentiated epidermoid cancer, and they formed cancer alveolus lesions in various sizes. Every portions corresponding to the cells located in peripheries of the cancer alveolus lesions gave distinct colored or decolorized traces formed by a protease, and the colored or decolorized traces formed by a protease at sites corresponding to the cancer cells of growing regions were particularly notable. Granular colored or decolorized traces formed by a protease were observed also at portions corresponding to focal inflammatory cell infiltration sites.

### 〈Severe epithelium dysplasia of oral mucosa〉

In a hematoxylin/eosin stained sample, severe epithelium dysplasia was recognized in epithelium, and hyperplasia of prickle cells and multiple stratification of basal cells were observed. In particular, hyperplasia and atypical cell growth were observed in basal cells. On the thin membrane, distinct colored or decolorized traces formed by a protease were recognized at portions corresponding to the hyperplastic prickle cell layer and the granulocyte layer, whereas only punctate colored or decolorized traces formed by a protease were recognized in the basal cell layer. These results suggest a high level of turnover of epithelial cells, whereas in basal cells infiltration into the epithelial connective tissues.

When the hyperplasia of prickle cells and the stratified basal cells were observed in magnification, colored or decolorized traces formed by a protease were recognized on portions corresponding to both of the cell layers, and reaction by a protease was observed at portions corresponding particularly to the prickle cells and stratified basal cells. On the other hand, reaction by a protease was weaker in basal cells in monolayer or bilayer compared with stratified basal cells. The stratified basal cells were deformed into a spindle shape, and hyperplastic and atypical cells were observed. Portions corresponding to the hyperplastic cells exhibiting growing tendency formed colored or decolorized traces by a protease on the thin membrane.

### 〈Measurement of protease activity in synovial liquid of rheumatism patients〉

About 20 µl of synovial liquid of a rheumatism patient was dropped on the thin membrane, and the membrane was incubated in a humidified box at 37°C for 1 to 3 hours. Then, distinct colored traces formed by a protease were observed at a peripheral circle of the applied circular spot formed on the thin membrane. In particular, when a thin membrane containing colloidal silver was used, especially remarkable colored or decolorized traces formed by a protease were observed.

### 〈Measurement of protease activity in periodontal diseases〉

Saliva and dental plaque on tooth surface were removed with cotton as completely as possible for simple elimination of moisture, and then a piece of Perio paper was inserted in gingival crevice and left stand for 90 seconds to allow the absorption of gingival crevicular fluid (about 5 to 10 µl) into the Perio paper. The piece of Perio paper was extracted with 150 µl of a buffer (50 mM Tris-HCl, pH 7.5, 10 mM CaCl₂, 0.2 M NaCl), and a protease was measured according to the same method as Example 6 using the extract as a sample solution. As a result, distinct colored or decolorized traces formed by a protease were observed along the peripheral circle of the applied circular spot on the thin membrane formed by the dropping of the gingival crevicular fluid.

### Comparative Example: Measurement of a protease utilizing an emulsion for radiography and results of measurement

As biosamples, gingiva, gingival crevicular fluid, and periodontal tissue samples collected from patients with a periodontal disease were affixed onto a microscope slide as about 5 µm frozen slices. Emulsion for radiography (KONICA CORPORATION) diluted with water was applied on the slices, and dried to form thin membranes. The thin membranes were placed in a humidified box, and incubated at 37°C for 16 hours to 14 days. Then, the membranes were stained with Amido Black or subjected to a monochromic development. As a result, almost no protease activity was detected. Trace of activity was observed in samples incubated for 14 days and then subjected to monochromic development. However, indication of the activity area was very ambiguous, and the membrane was concluded as practically useless as compared to the thin membranes of the present invention.

### Industrial Applicability

The methods of the present invention are characterized in that they enable accurate and convenient measurement of a protease derived from specific lesions localized in tissues or individual cells in tissues, and in addition, determination can be completed in a short period of time. Therefore, the methods of the present invention are useful for accurate diagnosis of malignancy of cancer cells such as infiltration and metastasis activities, degree of progress of a periodontal disease such as periodontitis, destructive pathological conditions of rheumatism, alveolar pyorrhea and the like. In addition, according to the methods of the present invention, protease activity can be measured by using an extremely small amount of a sample, and thin membranes after the measurement can be stored permanently as fixed preparations.

## Claims

1. A method for measuring an enzyme, which comprises the steps of:
(1) bringing a sample containing an enzyme into contact with a thin membrane comprising an organic compound which changes visible absorption upon reaction with the enzyme and comprising a hydrophilic polymer; and
(2) detecting a visible trace on the thin membrane formed by action of the enzyme.

2. A method for measuring an enzyme, which comprises the steps of:
(1) bringing one of two substantially continuous slices of a biosample into contact with a thin membrane comprising an organic compound which changes visible absorption upon reaction with the enzyme and comprising a hydrophilic polymer;
(2) detecting a visible trace formed by action of the enzyme on the thin membrane; and
(3) comparing the traces with a histopathological preparation prepared from the other slice.

3. A method for measuring an enzyme, which comprises the steps of:
(1) bringing one of two or more substantially continuous slices of a biosample into contact with a thin membrane comprising an organic compound which changes visible absorption upon reaction with the enzyme and comprising a hydrophilic polymer; (2) bringing the remaining slice(s) into contact with one or more thin membranes each comprising the organic compound, a hydrophilic polymer and an enzyme inhibitor;
(3) detecting a visible trace formed by action of the enzyme on each of the thin membranes; and
(4) comparing the trace on the thin membrane used in the step (1) with the trace on the thin membrane(s) used in the step (2).

4. A method for measuring an enzyme, which comprises the steps of:
(1) bringing one of several substantially continuous slices of a biosample into contact with a thin membrane comprising an organic compound which changes visible absorption upon reaction with the enzyme and comprising a hydrophilic polymer;
(2) bringing the remaining slice(s) into contact with one or more thin membranes each comprising the organic compound of the organic compound which is different from the organic compound contained in the thin membrane used in the step (1) and comprising a hydrophilic polymer;
(3) detecting a visible trace formed by action of the enzyme on each of the thin membranes; and
(4) comparing the trace on the thin membrane used in the step (1) with the trace on the thin membrane(s) used in the step (2).

5. A method for measuring an enzyme, which comprises the steps of:
(1) bringing one of two or more substantially continuous slices of a biosample into contact with a thin membrane comprising an organic compound which changes visible absorption upon reaction with the enzyme and comprising a hydrophilic polymer;
(2) bringing the remaining slice(s) into contact with one or more thin membranes comprising a substrate which is degradable by the enzyme;
(3) detecting a visible trace formed by action of the enzyme on each of the thin membranes; and
(4) comparing the trace on the thin membrane used in the step (1) with the trace on the thin membrane(s) used in the step (2).

6. A method for measuring an enzyme, which comprises the steps of:
(1) bringing a sample containing an enzyme into contact with a thin membrane comprising the following two layers:
layer (a) comprising one or more layers each of which comprises a thin membrane comprising an organic compound which changes visible absorption upon reaction with the enzyme and comprising a hydrophilic polymer, and
layer (b) comprising one or more layers each of which comprises a thin membrane comprising said organic compound, a hydrophilic polymer and an enzyme inhibitor;
(2) detecting a visible trace formed by action of the enzyme on the thin membrane; and
(3) comparing the trace on the layer (a) and the trace on the layer (b).

7. A method for measuring an enzyme, which comprises the steps of:
(1) bringing a sample containing an enzyme into contact with a thin membrane comprising two or more layers each of which comprises a thin membrane comprising an organic compound which changes visible absorption upon reaction with the enzyme and comprising a hydrophilic polymer, provided each layer contains a different kind of the organic compound;
(2) detecting a visible trace formed by action of the enzyme on the thin membrane; and
(3) comparing the traces on each of the layers.

8. A method for measuring an enzyme, which comprises the steps of:
(1) bringing a sample containing an enzyme into contact with a thin membrane comprising the following two layers:
layer (a) comprising one or more layers each of which comprises a thin membrane comprising an organic compound which changes visible absorption upon reaction with the enzyme and comprising a hydrophilic polymer; and
layer (b) comprising one or more layers each of which comprises a substrate degradable by the enzyme;
(2) detecting a visible trace formed by action of the enzyme on the thin membrane; and
(3) comparing the traces on the layer (a) and the traces on the layer (b).

9. The method according to any one of claims 1 to 8, wherein the thin membrane contains a hardening agent.

10. The method according to any one of claims 1 to 9, wherein the enzyme is a protease.

11. The method according to claim 10, wherein the enzyme is matrix metalloproteinase.

12. A method of diagnosing a disease in which an enzyme is involved, which comprises the steps of:
(1) bringing a biosample isolated or collected from a patient into contact with a thin membrane comprising an organic compound which changes visible absorption upon reaction with the enzyme and comprising a hydrophilic polymer, and wherein the thin membrane may optionally contain a hardening agent; and
(2) detecting a visible trace formed on the thin membrane to measure enzymatic activity in the biosample.

13. A thin membrane used for measurement of an enzyme, which comprises an organic compound which changes visible absorption upon reaction with the enzyme and a hydrophilic polymer, and wherein the thin membrane may optionally contain a hardening agent and/or an enzyme inhibitor.

14. A compound represented by the following general formula (1): 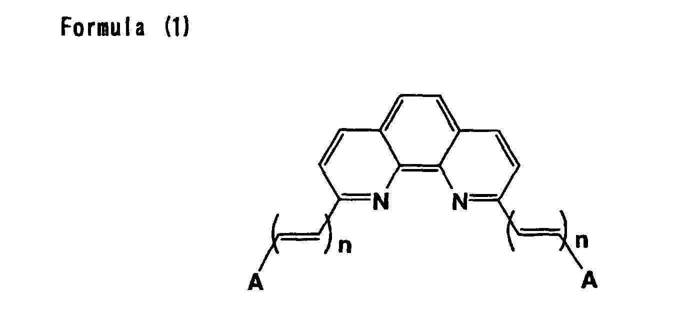 wherein the groups of A independently represent a substituted or unsubstituted aryl group or a substituted or unsubstituted heteroaryl group, and n independently represents 1 or 2.
